(19) Europäisches Patentamt
European Patent Office
Office européen des brevets

(11) Numéro de publication : **0 336 813 B1**

(12) **FASCICULE DE BREVET EUROPEEN**

(45) Date de publication du fascicule du brevet : **07.10.92 Bulletin 92/41**

(51) Int. Cl.[5] : **A61K 7/06,** A61K 31/505, A61K 31/17, A61K 31/40

(21) Numéro de dépôt : **89400822.6**

(22) Date de dépôt : **23.03.89**

(54) **Association de dérivés de pyrimidine et de dérivés d'urée et/ou d'allantoîne pour induire et stimuler la croissance des cheveux et diminuer leur chute.**

(30) Priorité : **31.03.88 LU 87188**

(43) Date de publication de la demande : **11.10.89 Bulletin 89/41**

(45) Mention de la délivrance du brevet : **07.10.92 Bulletin 92/41**

(84) Etats contractants désignés : **AT BE CH DE FR GB IT LI NL SE**

(56) Documents cités :
**EP-A- 0 277 428**
**FR-A- 2 160 286**
**NL-A- 7 414 311**
**US-A- 4 139 619**

(73) Titulaire : **L'OREAL**
**14, Rue Royale**
**F-75008 Paris (FR)**

(72) Inventeur : **Grollier, Jean-François**
**16-bis Boulevard Morland**
**F-75004 Paris (FR)**
Inventeur : **Rosenbaum, Georges**
**2, rue J.H. Mansart**
**F-92600 Asnières (FR)**

(74) Mandataire : **Casalonga, Axel et al**
**BUREAU D.A. CASALONGA - JOSSE**
**Morassistrasse 8**
**W-8000 München 5 (DE)**

**EP 0 336 813 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à l'association de dérivés de pyrimidine et de dérivés d'urée et/ou d'allantoïne en vue d'induire et de stimuler la croissance des cheveux et diminuer leur chute ainsi qu'aux compositions et aux procédés mis en oeuvre.

L'activité des follicules pileux est cyclique.

A la phase anagène active qui dure plusieurs années et au cours de laquelle les cheveux s'allongent, succède une phase de repos (télogène) de quelques mois. A la fin de cette période de repos, les cheveux tombent et un autre cycle recommence. La chevelure se renouvelle donc en permanence; sur les 100.000 à 150.000 cheveux que comporte une chevelure, à chaque instant, 10% environ sont au repos et seront donc remplacés en quelques mois.

Dans presque tous les cas, la chute des cheveux survient sur des sujets prédisposés génétiquement et elle atteint plus particulièrement les hommes. Il s'agit plus particulièrement de l'alopécie androgénétique ou androgénique.

Cette alopécie est une perturbation du renouvellement capillaire qui entraîne, dans un premier temps, une accélération de la fréquence des cycles aux dépens de la qualité des cheveux, puis de leur quantité. Il y a un appauvrissement progressif de la chevelure par régression des cheveux dits "terminaux" au stade de duvets. Des zones sont touchées préférentiellement : notamment les golfes temporaux ou frontaux chez l'homme et chez les femmes on constate une alopécie diffuse du vertex.

On a déjà proposé d'utiliser des composés tels que l'amino-6 dihydro-1,2 hydroxy-1 imino-2 pipéridino-4 pyrimidine ou "Minoxidil" dans des compositions permettant de réduire ou de supprimer l'effet de l'alopécie et d'induire et de stimuler la croissance des cheveux et diminuer leur chute.

La demanderesse a découvert maintenant, qu'en associant des dérivés de pyrimidine avec de l'urée ou ses dérivés, en particulier l'allantoïne, il était possible d'induire et de stimuler de façon améliorée la croissance des cheveux et d'avoir une action sur le freinage de leur chute.

Elle a également constaté que les compositions ainsi préparées présentaient une meilleure biodisponibilité cutanée, ainsi qu'une augmentation de l'efficacité.

Cette efficacité se caractérise en particulier par une activité supérieure par rapport aux dérivés de pyrimidine ou d'urée utilisés seuls.

Les compositions se caractérisent également par une action plus rapide et permettent d'utiliser les dérivés de pyrimidine dans des quantités beaucoup plus faibles.

Afin de déterminer l'efficacité ou la rapidité d'action des compositions de traitement de l'alopécie, on utilise généralement le trichogramme ou encore le phototrichogramme qui permet de déterminer, entre autres, le pourcentage de cheveux en phase anagène par rapport aux cheveux en phase télogène.

Un objet de l'invention est donc constitué par l'association d'urée ou de dérivés d'urée tels que l'allantoïne avec des dérivés de pyrimidine en vue d'induire ou de stimuler la croissance des cheveux et diminuer leur chute.

Un autre objet est constitué par les compositions cosmétiques et/ou pharmaceutiques contenant une telle association.

L'invention a également pour objet un procédé de traitement cosmétique.

Un objet de l'invention est également constitué par des dispositifs à plusieurs compartiments contenant l'association définie ci-dessus.

D'autres objets de l'invention apparaîtront à la lecture de la description et des exemples qui suivent.

L'association conforme à l'invention est essentiellement caractérisée par le fait qu'elle comprend :

a) un composant (A) contenant, dans un milieu physiologiquement acceptable, au moins un dérivé de pyrimidine répondant à la formule :

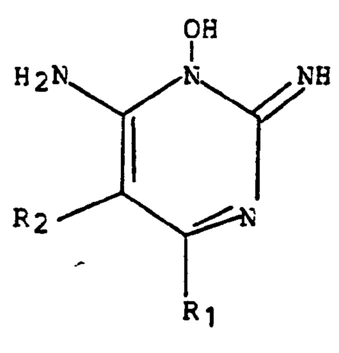

(I)

dans laquelle $R_1$ désigne le groupement

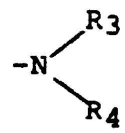

$R_3$, $R_4$, indépendamment l'un de l'autre, désignent hydrogène, un groupement alkyle, alcényle, alkylaryle, cycloalkyle, $R_3$ et $R_4$ pouvant également former un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylèneimine, morpholine, alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alcoxyalkyl, cycloalkyle, aryle, alkylaryle, arylalkyle, alkylarylalkyle, alcoxyarylalkyle, ou haloarylalkyle, ainsi que les sels d'addition d'acides physiologiquement acceptables; et

(b) un composant (B) contenant dans un milieu physiologiquement acceptable, au moins un composé répondant à la formule :

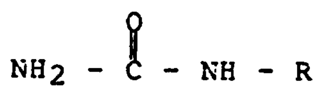

(II)

dans laquelle R désigne hydrogène ou le groupement de formule :

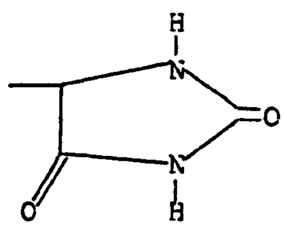

(III)

ainsi que les sels et les complexes de ces composés;

les composants (A) et (B) faisant partie d'une même composition ou étant destinés à être utilisés séparément, soit simultanément, soit de façon successive ou décalée dans le temps en vue d'induire et de stimuler la croissance des cheveux et diminuer leur chute.

Pour les composés de formule (I), les groupements alkyle ou alcoxy désignent de préférence un groupement alkyle ou alcoxy inférieur ayant 1 à 4 atomes de carbone; le groupement alcényle désigne de préférence un groupement alcényle inférieur ayant 2 à 5 atomes de carbone; le groupement aryle désigne de préférence phényle et le groupement cycloalkyle désigne de préférence un groupement ayant 4 à 6 atomes de carbone.

Les composés préférés de formule (I) sont plus particulièrement choisis parmi les composés dans lesquels $R_2$ désigne hydrogène et $R_1$ désigne un groupement :

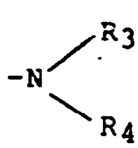 ,

dans lequel $R_3$ et $R_4$ désignent, indépendamment l'un de l'autre, hydrogène ou un groupement alkyle en $C_1$ à $C_4$ ou $R_3$ et $R_4$ forment un cycle pipéridinyle ou bien des groupements éthyle ainsi que leurs sels, tels que par exemple le sulfate.

On peut citer plus particulièrement le composé constitué par l'amino-6 dihydro-1,2 hydroxy-1 imino-2 diéthylamino-4 pyrimidine et le composé constitué par l'amino-6 dihydro-1,2 hydroxy-1 imino-2 pipéridino-4 pyrimidine, encore appelé "Minoxidil", ainsi que leurs sels tels que par exemple le sulfate.

Les composés de formule (II) ou leurs sels ou complexes particulièrement préférés sont choisis parmi

l'urée, l'allantoïne ou les complexes d'allantoïne tels que l'allantoïne-acide p-aminobenzoïque, l'allantoïne-acide galacturonique, l'allantoïne-acide polygalacturonique, l'allantoïne-acide glycyrrhétinique, l'allantoïne-acide ascorbique, l'allantoïne-biotine, l'allantoïne-acide pantothénique, l'allantoïne-acide succinique, l'allantoïne-acétylméthionine, l'allantoïne sodium ribonucléinate, l'allantoïne-sodium succinate, l'allantoïne-zinc undécylénate, l'allantoïne-calcium pantothénate; les complexes d'aluminium tels que l'allantoïnate d'hydroxyde de magnésium et d'aluminium l'allantoïnate d'hydroxychlorure d'aluminium, le p-aminobenzoate d'hydroxychlorure d'aluminium d'allantoïne et les complexes d'hydroxyde d'aluminium ou d'hydroxychlorure d'aluminium de biotine et de l'allantoïne, ceux de l'acide galacturonique ou polygalacturonique et de l'allantoïne, et ceux de l'acide pantothénique et de l'allantoïne.

Les dérivés de pyrimidine de formule (I) sont utilisés de préférence dans le composant (A) dans des proportions comprises entre 0,05 et 10% en poids et en particulier entre 0,05 et 5% en poids et préférentiellement entre 0,5 et 4% en poids.

Les dérivés de formule (II), leurs sels et leurs complexes sont de préférence utilisés dans des proportions comprises entre 0,1 et 30% en poids et de préférence entre 0,1 et 20% en poids et en particulier entre 0,1 et 10% en poids.

Les dérivés de pyrimidine de formule (I) lorsque les composants (A) et (B) sont dans la même composition, sont utilisés dans des proportions comprises entre 0,05 et 6% en poids par rapport au poids total de la composition et de préférence entre 0,1 et 5% en poids et en particulier entre 0,5 et 2% en poids.

Les dérivés de formule (II) ainsi que leurs sels et leurs complexes sont utilisés dans ce cas dans la composition unique dans des proportions comprises entre 0,05 et 30% en poids par rapport au poids total de la composition et de préférence entre 0,05 et 10% en poids et en particulier entre 0,05 et 5% en poids.

Le milieu physiologiquement acceptable pour les composants (A) et (B) est un milieu utilisable en pharmacie et en cosmétique et peut être constitué par de l'eau, un mélange d'eau et d'un ou plusieurs solvants organiques ou par un mélange de solvants organiques acceptables physiologiquement.

Ces compositions peuvent être pressurisées dans des dispositifs aérosols en présence d'un agent propulseur.

Les solvants plus particulièrement utilisables sont choisis parmi les alcools inférieurs en $C_1$-$C_4$, tels que l'alcool éthylique, l'alcool isopropylique, l'alcool tertiobutylique, les alkylèneglycols comme le propylèneglycol, les alkyléthers de mono- et de dialkylèneglycol tels que plus particulièrement le monoéthyléther d'éthylèneglycol, le monométhyléther de propylèneglycol et le monoéthyléther de diéthylèneglycol.

Les milieux physiologiquement acceptables peuvent être épaissis ou non. Pour les épaissir, on peut utiliser des agents épaississants ou gélifiants bien connus dans l'état de la technique tels que plus particulièrement des hétérobiopolysaccharides tels que la gomme de xanthane et les scléroglucanes, des dérivés de cellulose, des polymères acryliques réticulés ou non.

Les solvants, lorsqu'ils sont utilisés dans le milieu aqueux, sont présents de préférence dans des proportions comprises entre 1 et 80% en poids par rapport au poids total de la composition ou de chacun des composants (A) et (B).

Les épaississants, lorsqu'ils sont utilisés, sont présents de préférence dans des proportions comprises entre 0,1 et 5% en poids et en particulier entre 0,4 et 3% en poids par rapport au poids total de chacun des composants (A) et (B) lorsque ces composants sont utilisés de façon séparée ou par rapport au poids total de la composition contenant les composants (A) et (B).

Les compositions constituées, soit par l'un et/ou l'autres des composants (A) et (B) ou par la composition contenant les deux composants (A) et (B), peuvent également contenir tous autres adjuvants habituellement utilisés dans les compositions destinées à une application topique à utilisation cosmétique ou pharmaceutique et plus particulièrement des agents conservateurs, des agents complexants, des colorants, des agents alcalinisants ou acidifiants, des agents tensio-actifs, anioniques, cationiques, non-ioniques, amphotères ou leurs mélanges, des polymères anioniques, cationiques, non-ioniques ou amphotères ainsi que leurs mélanges. Le pH de ces compositions peut varier entre 4 et 9.

Dans le composant (A), les dérivés de pyrimidine de formule (I) peuvent être présents, soit sous forme dissoute dans le milieu physiologiquement acceptable ou alors en totalité ou partiellement en suspension dans ce milieu, en particulier sous forme micronisée. Dans ce cas, les dérivés de pyrimidine de formule (I) peuvent être présents sous forme de particules ayant une granulométrie inférieure à 80 microns et de préférence inférieure à 20 microns et en particulier inférieure à 5 microns.

Une forme de réalisation de l'invention consiste à utiliser l'association conforme à l'invention sous forme d'une composition contenant les composants (A) et (B).

Une autre forme préférée de l'invention consiste à conserver dans des dispositifs séparés les composants (A) et (B) et à préparer la composition contenant les dérivés de pyrimidine de formule (I) et les dérivés de formule (II) ou leurs complexes, de façon extemporanée tout juste avant l'emploi.

L'invention peut également consister à appliquer les composants (A) et (B) de façon séparée, soit de façon successive ou décalée dans le temps.

L'association conforme à l'invention peut être conditionnée dans ce cas dans un dispositif à plusieurs compartiments encore appelé "kit" ou nécessaire, dont un premier compartiment contient le composant (A) renfermant les dérivés de pyrimidine de formule (I) dans un milieu physiologiquement acceptable et le second compartiment contenant le composant (B), contenant dans un milieu physiologiquement acceptable les dérivés de formule (II) ou leurs sels ou complexes.

L'association conforme à l'invention permet de traiter thérapeutiquement la chute des cheveux en agissant plus particulièrement sur le dysfonctionnement des mécanismes biologiques à l'origine de la pousse des cheveux.

Le traitement peut consister à procéder à une application journalière de la composition contenant les composants (A) et (B) définie ci-dessus, soit préparée à l'avance, soit préparée au moment de l'emploi pendant une durée de quelques mois et ce à raison d'une application par jour.

L'association conforme à l'invention présente par ailleurs l'avantage d'être non irritante, hydratante, bactériostatique et kératinisante.

L'association conforme à l'invention permet également de traiter cosmétiquement les cheveux en vue de leur conférer une plus grande vigueur et un aspect meilleur.

L'invention a également pour objet l'utilisation de l'association telle que définie ci-dessus pour la préparation d'un médicament éventuellement appliqué en deux temps, destiné au traitement de l'alopécie et agissant notamment sur le dysfonctionnement du cycle pilaire.

Les exemples suivants sont destinés à illustrer l'invention.

EXEMPLE 1

On prépare une lotion de composition suivante :

| | | | |
|---|---|---|---|
| - Urée | | 3,0 | g |
| - Minoxidil | | 1,5 | g |
| - Ethanol/propylèneglycol (95/5) | qsp | 100,0 | g |

Cette composition est appliquée à raison de 2 grammes à la fréquence d'une fois par jour.

EXEMPLE 2

On prépare une lotion de composition suivante :

| | | | |
|---|---|---|---|
| - Allantoïne | | 0,23 | g |
| - Minoxidil | | 2,0 | g |
| - Propylèneglycol | | 20,0 | g |
| - Alcool éthylique | | 50,0 | g |
| - Conservateur | qs | | |
| - Eau | qsp | 100,0 | g |

Cette composition est appliquée de façon identique à l'exemple 1.

EXEMPLE 3

On conditionne en kit deux compositions (A) et (B) renfermant respectivement :

Composition (A) :

| | | | |
|---|---|---|---|
| - Minoxidil | | 2,0 | g |
| - Propylèneglycol | | 20,0 | g |
| - Alcool éthylique | | 50,0 | g |
| - Eau | qsp | 100,0 | g |

Composition (B) :

| | | | |
|---|---|---|---|
| - Urée | | 6,0 | g |
| - Propylèneglycol | | 20,0 | g |
| - Alcool éthylique | | 50,0 | g |
| - Eau | qsp | 100,0 | g |

On applique le mélange extemporané des compositions (A) et (B) une fois par jour à raison de 2 g de chacune des compositions.

EXEMPLE 4

On conditionne en kit deux compositions (A) et (B) renfermant respectivement :

Composition (A) :

| | | | |
|---|---|---|---|
| - Minoxidil de diamètre particulaire moyen inférieur à 2 microns | | 3,0 | g |
| - Acide polyacrylique réticulé PM = 3 millions, vendu sous la dénomination CARBOPOL 934 par la Société GOODRICH | | 1,0 | g |
| - Propylèneglycol | | 4,5 | g |
| - Amino-2 méthyl-2 propanol-1 | qs pH 7 | | |
| - Conservateur | qs | | |
| - Eau | qsp | 100,0 | g |

Composition (B) :

| | | | |
|---|---|---|---|
| - Allantoïne | | 0,60 | g |
| - Conservateur | qs | | |
| - Eau | qsp | 100,0 | g |

On applique le mélange extemporané des compositions (A) et (B) à raison de 1 gramme de mélange une fois par jour.

EXEMPLE 5

On conditionne en kit deux compositions (A) et (B) renfermant respectivement :

Composition (A) :

| | | | |
|---|---|---|---|
| - Minoxidil de diamètre particulaire moyen inférieur à 2 microns | | | 4,0 g |
| - Acide polyacrylique réticulé PM = 3 millions, vendu sous la dénomination CARBOPOL 934 par la Société GOODRICH | | | 1,0 g |
| - Propylèneglycol | | | 4,5 g |
| - Amino-2 méthyl-2 propanol-1 | qs | pH | 7 |
| - Conservateur | qs | | |
| - Eau | | qsp | 100,0 g |

Composition (B) :

| | | |
|---|---|---|
| - Urée | | 16,0 g |
| - Alcool éthylique | | 42,0 g |
| - Eau | qsp | 100,0 g |

Chaque jour, on applique les compositions en succession décalée dans le temps, le matin 1 g de la composition (A), le soir 1 g de la composition (B) ou inversement.

EXEMPLE 6

On conditionne en kit deux compositions (A) et (B) renfermant respectivement :

Composition (A) :

| | | |
|---|---|---|
| - Minoxidil | | 2,5 g |
| - Ethanol/propylèneglycol (95/5) | qsp | 100,0 g |

Composition (B) :

| | | | |
|---|---|---|---|
| - Allantoïne | | | 0,3 g |
| - Gomme de xanthane vendue sous la dénomination KELTROL T par la Société KELCO | | | 1,0 g |
| - Conservateur | qs | | |
| - Eau | | qsp | 100,0 g |

On applique les compositions en succession décalée dans le temps, chaque jour, 1 g de (A) le matin, puis 1 g de (B) le soir ou inversement.

EXEMPLE 7

On conditionne en kit deux compositions (A) et (B) renfermant respectivement :

Composition (A) :

| | | |
|---|---|---|
| - Amino-6 dihydro-1,2 hydroxy-1 imino-2 diéthylamino-4 pyrimidine | | 2,0 g |
| - Propylèneglycol | | 20,0 g |
| - Alcool éthylique | | 50,0 g |
| - Eau | qsp | 100,0 g |

Composition (B) :

| | | |
|---|---|---|
| - Allantoïne | | 0,3 g |
| - Propylèneglycol | | 20,0 g |
| - Alcool éthylique | | 50,0 g |
| - Conservateur | qs | |
| - Eau | qsp | 100,0 g |

Chaque jour, on applique les compositions en succession décalée dans le temps, le matin 1 g de la composition (A), le soir 1 g de la composition (B) ou inversement, sur les zones alopéciques du cuir chevelu.

EXEMPLE 8

On prépare la lotion de composition suivante, qu'on applique sur les zones alopéciques du cuir chevelu :

| | | |
|---|---|---|
| - Minoxidil | | 0,54 g |
| - Urée | | 5,43 g |
| - Propylèneglycol | | 22,8 g |
| - Alcool éthylique | | 55,1 g |
| - Eau | qsp | 100,0 g |

EXEMPLE 9

On prépare une lotion de composition suivante :

| | | |
|---|---|---|
| - Minoxidil | | 0,5 g |
| - Allantoïne | | 0,5 g |
| - Alcool éthylique/eau (25/75) en poids | qsp | 100,0 g |

EXEMPLE 10

On prépare une lotion de composition suivante :

| | | | |
|---|---|---|---|
| - Minoxidil | | 1,5 | g |
| - Complexe d'allantoïne et d'acide glycyrrhétinique vendu sous la dénomination ALGLYCERA par la Société ICI AMERICAS | | 0,65 | g |
| - Propylène glycol | | 22,8 | g |
| - Alcool éthylique | | 55,1 | g |
| - Eau | qsp | 100,0 | g |

EXEMPLE 11

On prépare une lotion de composition suivante :

| | | | |
|---|---|---|---|
| - Minoxidil | | 2,0 | g |
| - Complexe d'allantoïne/acide paraamino-benzoïque vendu sous la dénomination ALPABA par la Société ICI AMERICAS | | 1,25 | g |
| - Alcool éthylique/eau (25/75) en poids | qsp | 100,0 | g |

EXEMPLE 12

On prépare une lotion de composition suivante :

| | | | |
|---|---|---|---|
| - Minoxidil | | 3,0 | g |
| - Complexe d'allantoïne/acide paraamino-benzoïque vendu sous la dénomination ALPABA par la Société ICI AMERICAS | | 2,5 | g |
| - Propylène glycol | | 22,8 | g |
| - Alcool éthylique | | 55,1 | g |
| - Eau | qsp | 100,0 | g |

EXEMPLE 13

On prépare une lotion de composition suivante :

| | | | |
|---|---|---|---|
| - Minoxidil | | 0,5 | g |
| - Complexe d'allantoïne et d'acide ascorbique (1/1 en mole) | | 1,0 | g |
| - Alcool éthylique/eau (25/75) en poids | qsp | 100,0 | g |

EXEMPLE 14

On prépare une lotion de composition suivante :

| | | | |
|---|---|---|---|
| - Complexe d'allantoïne/biotine (43/57) en poids | | 4,0 | g |
| - Minoxidil | | 0,5 | g |
| - Alcool éthylique/eau (25/75) en poids | qsp | 100,0 | g |

EXEMPLE 15

On prépare une lotion de composition suivante :

| | | | |
|---|---|---|---|
| - Minoxidil | | 0,5 | g |
| - Complexe allantoïne/pantothénate de calcium (25/75) en poids | | 2,0 | g |
| - Alcool éthylique/eau (25/75) en poids | qsp. | 100,0 | g |

**Revendications**

**Revendications pour les Etats contractants suivants : BE, CH, DE, FR, GB, IT, LI, NL, SE, AT**

1. Association destinée à induire et à stimuler la croissance des cheveux et diminuer leur chute, caractérisée par le fait qu'elle comprend :
   a) un composant (A) contenant dans un milieu physiologiquement acceptable, au moins un dérivé de pyrimidine répondant à la formule :

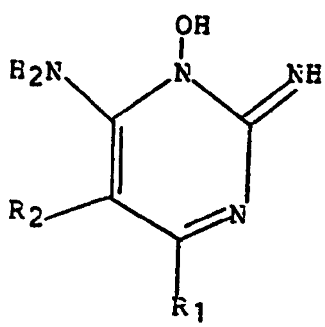

(I)

dans laquelle $R_1$ désigne le groupement

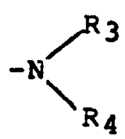

dans lequel $R_3$ et $R_4$, indépendamment l'un de l'autre désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine, et alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alcoxyalkyl, cycloalkyle, aryle, alkylaryle, arylalkyle, alkylarylalkyle, alcoxyarylalkyle ou haloarylalkyle, ainsi que les sels d'addition d'acides physiologiquement acceptables; et

b) un composant (B) contenant dans un milieu physiologiquement acceptable, au moins un dérivé répondant à la formule :

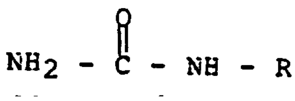

(II)

dans laquelle R désigne hydrogène ou un groupement de formule :

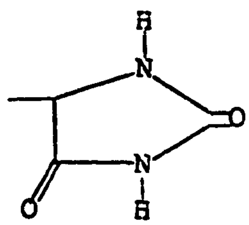

(III)

ainsi que les sels et les complexes physiologiquement acceptables de ces composés;

les composants (A) et (B) faisant partie d'une même composition ou étant destinés à être utilisés séparément, soit simultanément, soit de façon successive ou décalée dans le temps sur les cheveux et le cuir chevelu.

2. Association selon la revendication 1, caractérisée par le fait que le composé de formule (I) est constitué par l'amino-6 dihydro-1,2 hydroxy-1 imino-2 pipéridino-4 pyrimidine ou "Minoxidil".

3. Association selon la revendication 1, caractérisée par le fait que le composé de formule (I) est constitué par l'amino-6 dihydro-1,2 hydroxy-1 imino-2 diéthylamino-4 pyrimidine.

4. Association selon la revendication 1, caractérisée par le fait que les composés de formule (II) sont choisis parmi l'urée, l'allantoïne, l'allantoïne-acide p-aminobenzoïque, l'allantoïne-acide galacturonique, l'allantoïne-acide polygalacturonique, l'allantoïne-acide glycyrrhétinique, l'allantoïne-acide ascorbique, l'allantoïne-biotine, l'allantoïne-acide pantothénique, l'allantoïne-acide succinique, l'allantoïne-acétylméthionine, l'allantoïne-sodium ribonucléinate, l'allantoïne-sodium succinate, l'allantoïne-zinc undécylénate, l'allantoïne-calcium pantothénate; l'allantoïne-d'hydroxyde de magnésium et d'aluminium, l'allantoïnate d'hydroxychlorure d'aluminium, le p-aminobenzoate d'hydroxychlorure d'aluminium d'allantoïne et les complexes d'hydroxyde d'aluminium ou d'hydroxychlorure d'aluminium de la biotine et de l'allantoïne, les complexes d'acide galacturonique ou polygalacturonique et de l'allantoïne, les complexes d'acide pantothénique et d'allantoïne.

5. Association selon l'une quelconque des revendications 1 à 4, caractérisée par le fait que le dérivé de pyrimidine de formule (I) est utilisé dans le composant (A) dans des proportions comprises entre 0,05 et 10% en poids, et de préférence entre 0,05 et 5% en poids, et que le dérivé de formule (II), ses sels ou ses complexes est (sont) présent(s) dans le composant (B) dans des proportions comprises entre 0,1 et 30% en poids et de préférence entre 0,1 et 20% en poids.

6. Association selon l'une quelconque des revendications 1 à 4, caractérisée par le fait que les composants (A) et (B) sont présents dans la même composition et comprennent le dérivé de pyrimidine de formule (I) dans des proportions comprises entre 0,05 et 6% en poids par rapport au poids total de la composition et de préférence entre 0,1 et 5% en poids et que le dérivé de formule (II), ses sels ou ses complexes est (sont) présent(s) dans des proportions comprises entre 0,05 et 30% en poids par rapport au poids total de la composition et de préférence entre 0,05 et 10% en poids par rapport au poids total de la composition.

7. Association selon l'une quelconque des revendications 1 à 6, caractérisée par le fait que le milieu physiologiquement acceptable est constitué par de l'eau, un mélange d'eau et d'un ou plusieurs solvants organiques ou par un mélange de solvants organiques pharmaceutiquement ou cosmétiquement acceptables.

8. Association selon la revendication 7, caractérisée par le fait que les solvants sont choisis parmi les alcools inférieurs en $C_1$-$C_4$, les alkylène-glycols, les alkyléthers de mono- et de dialkylène-glycol.

9. Association selon l'une quelconque des revendications 1 à 8, caractérisée par le fait que l'un au moins des milieux physiologiquement acceptables des composants (A) et (B) est épaissi au moyen d'agents épaississants et/ou gélifiants.

10. Association selon l'une quelconque des revendications 1 à 9, caractérisée par le fait que l'un au moins des composants (A) et/ou (B) contient également au moins un adjuvant cosmétiquement ou pharmaceutiquement acceptable choisi parmi les agents conservateurs, les agents complexants, des colorants, des agents alcalinisants ou acidifiants, des agents tensio-actifs anioniques, cationiques, non-ioniques ou amphotères ainsi que leurs mélanges, des polymères anioniques, cationiques, non-ioniques ou amphotères ainsi que leurs mélanges.

11. Dispositif à plusieurs compartiments ou "kit" ou nécessaire, caractérisé par le fait qu'il comprend, dans un premier compartiment, le composant (A) contenant au moins dans un milieu physiologiquement acceptable un dérivé de pyrimidine répondant à la formule (I) et dans un second compartiment, le composant (B) contenant dans un milieu physiologiquement acceptable, au moins un dérivé de formule (II), ses sels ou ses complexes.

12. Association selon l'une quelconque des revendications 1 à 10 pour son application comme médicament dans le traitement de l'alopécie.

13. Utilisation de l'association selon l'une quelconque des revendications 1 à 10 pour la préparation d'un médicament destiné à traiter l'alopécie.

14. Procédé de traitement cosmétique des cheveux ou du cuir chevelu comprenant l'application de l'association définie dans l'une quelconque des revendications 1 à 10.

## Patentansprüche

### Patentansprüche für folgende Vertragsstaaten : BE, CH, DE, FR, GB, IT, LI, NL, SE, AT

1. Assoziat zum Induzieren und Stimulieren des Haarwuchses und zum Vermindern des Haarausfalles, dadurch **gekennzeichnet,** daß es umfaßt:

   a) einen Bestandteil (A), enthaltend in einem physiologisch verträglichen Milieu mindestens ein Pyrimidinderivat der Formel:

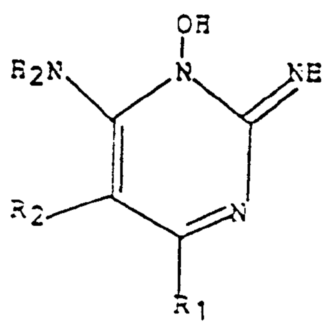

(I)

worin $R_1$ die

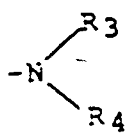

Gruppe darstellt, worin $R_3$ und $R_4$, unabhängig voneinander, Wasserstoff, eine Alkyl-, Alkenyl-, Alkylaryl-, Cycloalkylgruppe bedeuten, wobei $R_3$ und $R_4$ auch einen Heterocyclus mit dem Stickstoffatom, an das sie gebunden sind, bilden können, ausgewählt aus Aziridinyl, Azetidinyl, Pyrrolidinyl-, Piperidinyl-, Hexahydroazepinyl-, Heptamethylenimin-, Octamethylenimin-, Morpholin-und Niedrigalkyl-4-piperazidinylgruppen, wobei die heterozyklischen Gruppen an den Kohlenstoffatomen durch eine bis drei Niedrigalkyl-, Hydroxy- oder Alkoxygruppen substituiert sein können, und worin die $R_2$- Gruppe unter einem Wasserstoffatom, einer Alkyl-, Alkenyl-, Alkoxyalkyl-, Cycloalkyl-, Aryl-, Alkylaryl-, Arylalkyl-, Alkylarylalkyl-, Alkoxiarylalkyl- oder Haloarylalkylgruppe ausgewählt ist, sowie die physiologisch verträglichen Säureadditionssalze davon; und b) einen Bestandteil (B), enthaltend in einem physiologisch verträglichen Milie meindestens ein Derivat der Formel:

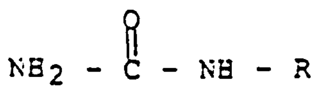

(II)

worin R Wasserstoff oder eine Gruppe der Formel bedeutet:

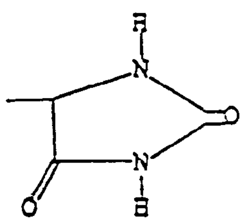

(III)

sowie die physiologisch verträglichen Salze und Komplexe dieser Verbindungen,

wobei die Bestandteile (A) und (B) Teile ein und derselben Zusammensetzung darstellen oder dazu bestimmt sind, auf den Haaren und behaarter Haut getrennt verwendet zu werden, entweder gleichzeitig oder aufeinanderfolgend oder nach einem Zeitablauf.

2. Assoziat gemäß Anspruch 1,
dadurch **gekennzeichnet,** daß die Verbindung der Formel (I) aus Amino-6-dihydro-1,2-hydroxy-1-imino-2-piperidino-4-pyrimidin oder "Minoxidil", zusammengesetzt ist.

3. Assoziat gemäß Anspruch 1,
dadurch **gekennzeichnet,** daß die Verbindung der Formel (I) aus Amino-6-dihydro-1,2-hydroxy-1-imino-2-diethylamino-4-pyrimidin zusammengesetzt ist.

4. Assoziat gemäß Anspruch 1,
dadurch **gekennzeichnet,** daß die Verbindungen der Formel (II) ausgewählt sind aus Harnstoff, Allantoin, Allantoin-p-aminobenzoesäure, Allantoin-Galacturonsäure, Allantoin-Polyqalacturonsäure, Allantoin-Glycyrrhetinsäure, Allantoin-Ascorbinsäure, Allantoin-Biotin, Allantoin-Pantothensäure, Allantoin-Bernsteinsäure, Allantoin-Acetylmethionin, Allantoin-Natriumribonucleinat, Allantoin-Natriumsuccinat, Allantoin-Zinkundecylenat, Allantoin-Calciumpantothenat, Allantoin-Magnesium- und Aluminiumhydroxid, Allantoinat von Aluminiumhydroxychlorur, dem p-Aminobenzoat des Aluminiumhydroxychlorurs von Allantoin und den Aluminiumhydroxid- oder -hydroxychlorurkomplexen des Biotins und Allantoins, den Galacturon- oder Polygalacturonsäurekomplexen des Allantoins und den Pantothensäurekomplexen des Allantoins.

5. Assoziat gemäß eines jeden der Ansprüche 1 bis 4,
dadurch **gekennzeichnet,** daß das Pyrimidinderivat der Formel (I) im Bestandteil (A) in Mengen von 0,05 bis 10, vorzugsweise 0,05 bis 5, Gewichtsprozent eingesetzt wird, und daß das Derivat der Formel (II),

seine Salze oder seine Komplexe im Bestandteil (B) in Mengen von 0,1 bis 30, vorzugsweise 0,1 bis 20, Gewichtsprozent vorliegt (vorliegen).

6. Assoziat gemäß jedem der Ansprüche 1 bis 4, dadurch **gekennzeichnet,** daß die Bestandteile (A) und (B) in derselben Zusammensetzung vorliegen und das Pyrimidinderivat der Formel (I) in Mengen von 0,05 bis 6, vorzugsweise 0,1 bis 5, Gewichtsprozent, bezogen auf Gesamtgewicht der Zusammensetzung, enthalten ist und daß das Derivat der Formel (II), seine Salze oder seine Komplexe in Mengen von 0,05 bis 30, vorzugsweise 0,05 bis 10, Gewichtsprozent, bezogen auf Gesamtgewicht der Zusammensetzung, vorliegt (vorliegen).

7. Assoziat gemäß eines jeden der Ansprüche 1 bis 6, dadurch **gekennzeichnet,** daß das physiologisch verträgliche Milieu aus Wasser, einer Mischung aus Wasser und eines oder mehrerer organischer Lösungsmittel oder aus einer mischung aus pharmazeutisch oder kosmetisch verträglichen organischen Lösungsmitteln zusammengesetzt ist.

8. Assoziat gemäß Anspruch 7, dadurch **gekennzeichnet,** daß die Lösungsmittel aus $C_1$-$C_4$- Niedrigalkoholen, Alkylenglycolen, Alkylethern von Mono- und Dialkylenglycol ausgewählt sind.

9. Asosziat gemäß eines jeden der Ansprüche 1 bis 8, dadurch **gekennzeichnet,** daß mindestens eines der physiologisch verträglichen Medien der Bestandteile (A) und (B) durch Verdickungsmittel und/oder Geliermittel verdickt ist.

10. Assoziat gemäß eines jeden der Ansprüche 1 bis 9, dadurch **gekennzeichnet,** daß mindestens einer der Bestandteile (A) und/oder (B) auch mindestens einen kosmetisch oder pharmazeutisch verträglichen Hilfsstoff enthält, ausgewählt aus Konservierungs-, Komplexierungs-, Färbe-, alkalisch oder sauer machenden Mitteln, anionischen, kationischen, nicht-ionischen oder amphoteren oberflächenaktiven Mitteln sowie deren Mischungen, anionischen, kationischen, nicht-ionischen oder amphoteren Polymeren sowie deren Mischungen.

11. Vorrichtungen aus mehreren Teilen oder "Kit" oder Necessaire, dadurch **gekennzeichnet,** daß sie in einem ersten Teil den Bestandteil (A), umfassend mindestens in einem physiologisch verträglichen Milieu ein Pyrimidinderivat der Formel (I), und in einem zweiten Teil den Bestandteil (B), umfassend in einem physiolögisch verträglichen Milieu mindestens ein Derivat der Formel (II), seine Salze oder seine Komplexe, enthalten.

12. Assoziat gemäß eines jeden der Ansprüche 1 bis 10, zur Verwendung als Medikament bei der Behandlung von Alopezie.

13. Verwendung des Assoziats gemäß eines jeden der Ansprüche 1 bis 10 zur Herstellung eines Medikaments zur Behandlung von Alopezie.

14. Verfahren zur kosmetischen Behandlung der Haare oder behaarter Haut, wobei man das Assoziat gemäß eines jeden der Ansprüche 1 bis 10 aufbringt.

## Claims

### Claims for the following Contracting States : BE, CH, DE, FR, GB, IT, LI, NL, SE, AT

1. Combination intended for inducing and stimulating hair growth and reducing hair loss, characterised in that it comprises:

   a) a component (A) containing, in a physiologically acceptable medium, at least one pyrimidine derivative corresponding to the formula:

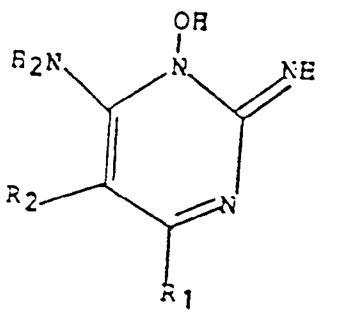

(I)

in which $R_1$ denotes the group

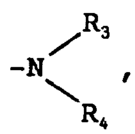

in which $R_3$ and $R_4$. independently of one another, denote hydrogen or an alkyl, alkenyl, alkylaryl or cycloalkyl group, $R_3$ and $R_4$ can also form a heterocycle with the nitrogen atom to which they are linked, chosen from aziridinyl, azetidinyl, pyrrolidinyl, piperidyl, hexahydroazepinyl, heptamethylenimine, octamethylenimine, morpholine and 4-(lower alkyl)piperazinyl groups, it being possible for the heterocyclic groups to be substituted on the carbon atoms with one to three lower alkyl, hydroxyl or alkoxy groups; the group $R_2$ is chosen from a hydrogen atom and an alkyl, alkenyl, alkoxyalkyl, cycloalkyl, aryl, alkylaryl, arylalkyl, alkylarylalkyl, alkoxyarylalkyl or haloarylalkyl group; as well as the addition salts with pharmaceutically acceptable acids; and

b) a component (B) containing, in a physiologically acceptable medium, at least one derivative corresponding to the formula:

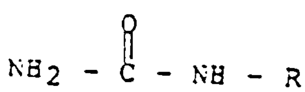

(II)

in which R denotes hydrogen or a group of formula:

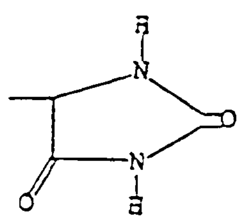

(III)

as well as the physiologically acceptable salts and complexes of these compounds;

the components (A) and (B) forming part of one and the same composition or being intended for use separately, either simultaneously or successively or separated by an interval of time, on the hair and the scalp.

2. Combination according to Claim 1, characterised in that the compound of formula (I) consists of 6-amino-1,2-dihydro-1-hydroxy-2-imino-4-piperidinopyrimidine or "minoxidil".

3. Combination according to Claim 1, characterised in that the compound of formula (I) consists of 6-amino-1,2-dihydro-1-hydroxy-2-imino-4-(diethylamino)pyrimidine.

4. Combination according to Claim 1, characterised in that the compounds of formula (II) are chosen from urea, allantoin, allantoin-p-aminobenzoic acid, allantoin-galacturonic acid, allantoin-polygalacturonic acid,

allantoin-glycyrrhetic acid, allantoin-ascorbic acid, allantoin-biotin, allantoin-pantothenic acid, allantoin-succinic acid, allantoin-acetylmethionine, allantoin-sodium ribonucleinate, allantoin-sodium succinate, allantoin-zinc undecylenate, allantoin-calcium pantothenate; aluminium magnesium hydroxide allantoinate, chlorohydroxyaluminium allantoinate, allantoin chlorohydroxyaluminium p-aminobenzoate and the aluminium hydroxide or aluminium hydroxychloride complexes of biotin and allantoin, the complexes of galacturonic or polygalacturonic acid and allantoin, the complexes of pantothenic acid and allantoin.

**5.** Combination according to any one of Claims 1 to 4, characterised in that the pyrimidine derivative of formula (I) is used in the component (A) in proportions of between 0.05 and 10 % by weight, and preferably between 0.05 and 5 % by weight, and in that the derivative of formula (II), its salts or its complexes is/are present in the component (B) in proportions of between 0.1 and 30 % by weight, and preferably between 0.1 and 20 % by weight.

**6.** Combination according to any one of Claims 1 to 4, characterised in that the components (A) and (B) are present in the same composition and comprise the pyrimidine derivative of formula (I) in proportions of between 0.05 and 6 % by weight relative to the total weight of the composition, and preferably between 0.1 and 5 % by weight, and in that the derivative of formula (II), its salts or its complexes is/are present in proportions of between 0.05 and 30 % by weight relative to the total weight of the composition, and preferably between 0.05 and 10 % by weight relative to the total weight of the composition.

**7.** Combination according to any one of Claims 1 to 6, characterised in that the physiologically acceptable medium consists of water or a mixture of water and one or more organic solvents, or of a mixture of pharmaceutically or cosmetically acceptable organic solvents.

**8.** Combination according to Claim 7, characterised in that the solvents are chosen from $C_1$-$C_4$ lower alcohols, alkylene glycols, and mono- and dialkylene glycol alkyl ethers.

**9.** Combination according to any one of Claims 1 to 8, characterised in that at least one of the physiologically acceptable media of the components (A) and (B) is thickened by means of thickening and/or gelling agents.

**10.** Combination according to any one of Claims 1 to 9, characterised in that at least one of the components (A) and/or (B) also contains at least one cosmetically or pharmaceutically acceptable adjuvant, chosen from preservatives, complexing agents, colorants, alkalinising or acidifying agents, anionic, cationic, nonionic or amphoteric surfactants as well as mixtures thereof, anionic, cationic, nonionic or amphoteric polymers as well as mixtures thereof.

**11.** Multi-compartment device or kit or outfit, characterised in that it comprises, in a first compartment, the component (A) containing at least one pyrimidine derivative corresponding to the formula (I) in a physiologically acceptable medium, and in a second compartment, the component (B) containing at least one derivative of formula (II), its salts or its complexes in a physiologically acceptable medium.

**12.** Combination according to any one of Claims 1 to 10 for application as a medicinal product in the treatment of alopecia.

**13.** Use of the combination according to any one of Claims 1 to 10 for the preparation of a medicinal product intended for treating alopecia.

**14.** Process for cosmetic treatment of the hair or the scalp, comprising the application of the combination defined in any one of Claims 1 to 10.